(19) Europäisches Patentamt
European Patent Office
Office européen des brevets

(11) EP 4 796 101 A1

(12) **EUROPEAN PATENT APPLICATION**
published in accordance with Art. 153(4) EPC

(43) Date of publication:
**26.08.2026 Bulletin 2026/35**

(21) Application number: **24879486.9**

(22) Date of filing: **18.09.2024**

(51) International Patent Classification (IPC):
***A61K 8/26*** *(2006.01)* ***A61K 8/34*** *(2006.01)*
***A61K 8/63*** *(2006.01)* ***A61Q 15/00*** *(2006.01)*

(52) Cooperative Patent Classification (CPC):
**A61K 8/26; A61K 8/34; A61K 8/63; A61Q 15/00**

(86) International application number:
**PCT/JP2024/033226**

(87) International publication number:
**WO 2025/084059 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.10.2023 JP 2023180491**

(71) Applicant: **Mandom Corporation**
**Osaka-shi, Osaka 540-8530 (JP)**

(72) Inventors:
- **NAKAGAWA, Atsushi**
  **Osaka-shi, Osaka 540-8530 (JP)**
- **INADA, Koki**
  **Osaka-shi, Osaka 540-8530 (JP)**
- **NAGAMATSU, Moriaki**
  **Osaka-shi, Osaka 540-8530 (JP)**

(74) Representative: **TBK**
**Bavariaring 4-6**
**80336 München (DE)**

(54) **ANTIPERSPIRANT COMPOSITION**

(57) Provided is an antiperspirant composition capable of forming a uniform film on the skin. The antiperspirant composition of the present invention contains ammonium glycyrrhizate (component A), aluminum chlorohydrate (component B), ethanol (component C), and water (component D), in which a content of the component A is 0.05 mass% or more and 1.0 mass% or less, a content of the component B is 3.0 mass% or more and 30.0 mass% or less, a mass ratio of the content of the component C to the content of the component A is 20.0 or more, a mass ratio of the content of the component D to the content of the component A is 10.0 or more, and a mass ratio of the content of the component D to the content of the component B is 0.8 or more.

EP 4 796 101 A1

## Description

### TECHNICAL FIELD

The present invention relates to an antiperspirant composition.

### BACKGROUND ART

Body odor such as armpit odor is generated when sweat and sebum are mixed, and decomposed by skin resident bacteria. Therefore, for preventing body odor, it is effective to inhibit perspiration that causes odor, and an antiperspirant composition containing an antiperspirant component is used.

An antiperspirant composition containing aluminum chlorohydrate as an antiperspirant component is known (For example, Patent Document 1). In the antiperspirant composition containing aluminum chlorohydrate, a film is formed on the skin, and the formed film blocks perspiration glands to exhibit an antiperspirant effect.

### PRIOR ART DOCUMENT

### PATENT DOCUMENT

Patent Document 1: JP 2003-095906 A

### SUMMARY OF THE INVENTION

### PROBLEMS TO BE SOLVED BY THE INVENTION

The present inventors have found that a conventional antiperspirant composition containing aluminum chlorohydrate has a problem that a film after the antiperspirant composition is applied onto the skin and dried is easily cracked, and it is difficult to form a uniform film on the skin.

Therefore, an object of the present invention is to provide an antiperspirant composition capable of forming a uniform film on the skin.

### MEANS FOR SOLVING THE PROBLEM

The present invention provides an antiperspirant composition containing a component (A), a component (B), a component (C), and a component (D), in which a content of the component (A) is 0.05 mass% or more and 1.0 mass% or less, a content of the component (B) is 3.0 mass% or more and 30.0 mass% or less, a mass ratio of the content of the component (C) to the content of the component (A) is 20.0 or more, a mass ratio of the content of the component (D) to the content of the component (A) is 10.0 or more, and a mass ratio of the content of the component (D) to the content of the component (B) is 0.8 or more.

The component (A): ammonium glycyrrhizate
The component (B): aluminum chlorohydrate
The component (C): ethanol
The component (D): water

### EFFECT OF THE INVENTION

The antiperspirant composition of the present invention contains a specific component (A), a specific component (B), a specific component (C), and a specific component (D). In the antiperspirant composition of the present invention, the content of the component (A) is 0.05 mass% or more and 1.0 mass% or less, the content of the component (B) is 3.0 mass% or more and 30.0 mass% or less, the mass ratio of the content of the component (C) to the content of the component (A) is 20.0 or more, the mass ratio of the content of the component (D) to the content of the component (A) is 10.0 or more, and the mass ratio of the content of the component (D) to the content of the component (B) is 0.8 or more. The antiperspirant composition of the present invention, which has the above-described configuration, thus can form a uniform film on the skin.

MODE FOR CARRYING OUT THE INVENTION

Hereinafter, the present invention will be described in detail.

An antiperspirant composition of the present invention contains ammonium glycyrrhizate, aluminum chlorohydrate, ethanol, and water.

In the present specification, the "ammonium glycyrrhizate" may be referred to as a "component (A)".

In the present specification, the "aluminum chlorohydrate" may be referred to as a "component (B)".

In the present specification, the "ethanol" may be referred to as a "component (C)".

In the present specification, the "water" may be referred to as a "component (D)".

Thus, the antiperspirant composition of the present invention contains the component (A), the component (B), the component (C), and the component (D).

In the antiperspirant composition of the present invention, the content of the component (A) is 0.05 mass% or more and 1.0 mass% or less, and the content of the component (B) is 3.0 mass% or more and 30.0 mass% or less.

In the antiperspirant composition of the present invention, the mass ratio of the content of the component (C) to the content of the component (A) (content of component (C)/content of component (A)) is 20.0 or more, the mass ratio of the content of the component (D) to the content of the component (A) (content of component (D)/content of component (A)) is 10.0 or more, and the mass ratio of the content of the component (D) to the content of the component (B) (content of component (D)/content of component (B)) is 0.8 or more.

The antiperspirant composition of the present invention, which has the above-described configuration, thus can form a uniform film on the skin. That is, the antiperspirant composition of the present invention, which has the above-described configuration, thus is capable of enhancing the uniformity of a film formed on the skin.

The present inventors have found, in an antiperspirant composition containing the component (B), a combination of types and mass ratios of components which enables a uniform film to be formed on the skin.

In particular, the present inventors have found that the uniformity of the film formed on the skin can be enhanced to some extent by combining the component (A) with the component (B). However, in the case where the component (A) and the component (B) are simply mixed with water (component (D)), the component (B) is precipitated in the antiperspirant composition, so that a uniform film cannot be formed on the skin. The present inventors have conducted intensive studies, and resultantly found that a uniform film can be formed on the skin by further combining ethanol (component (C)) with the components (A), (B) and (D) and using the components (A) to (D) at a specific mass ratio.

In the antiperspirant composition of the present invention, the components (A) and (B) can be allowed to exist in a good dissolved state in the antiperspirant composition. A film after the antiperspirant composition of the present invention is applied onto the skin and dried is hardly cracked. Thus, the antiperspirant composition of the present invention can form a uniform film on the skin. The antiperspirant composition of the present invention, which can form a uniform film on the skin, thus effectively exhibits an antiperspirant effect.

The antiperspirant composition of the present invention may contain components other than the components (A) to (D).

In the present specification, the content of each component means the sum of the contents of all the components contained in the antiperspirant composition. For example, the content of the component (A) means the sum of the contents of all the components (A) in the antiperspirant composition.

(Component (A))

The component (A) is ammonium glycyrrhizate (ammonium glycyrrhizinate). By using the component (A) in combination with the component (B), cracking of the film is effectively suppressed, and the effect of the present invention can be exhibited. Only one kind of the component (A) may be used, or two or more kinds thereof may be used in combination.

Examples of the component (A) include monoammonium glycyrrhizate (monoammonium glycyrrhizinate) and diammonium glycyrrhizate.

From the viewpoint of more effectively exhibiting the effect of the present invention, the component (A) preferably contains monoammonium glycyrrhizate.

In the present specification, the "monoammonium glycyrrhizate" may be referred to as a "component (A1)".

Therefore, the component (A) preferably includes the component (A1). The antiperspirant composition of the present invention preferably contains the component (A1).

From the viewpoint of exhibiting the effect of the present invention, the content of the component (A) is 0.05 mass% or more and 1.0 mass% or less per 100 mass% of the antiperspirant composition of the present invention.

The content of the component (A) is preferably 0.1 mass% or more, and more preferably 0.2 mass% or more, and preferably 0.8 mass% or less, and more preferably 0.6 mass% or less, per 100 mass% of the antiperspirant composition of the present invention. When the content of the component (A) is equal to or more than the lower limit and equal to or less

than the upper limit, the effect of the present invention can be further enhanced. In particular, when the content of the component (A) is equal to or less than the upper limit, generation of a precipitate in the antiperspirant composition can be effectively suppressed.

The content of the component (A1) is preferably 0.05 mass% or more, more preferably 0.1 mass% or more, and still more preferably 0.2 mass% or more, and preferably 1.0 mass% or less, more preferably 0.8 mass% or less, and still more preferably 0.6 mass% or less, per 100 mass% of the antiperspirant composition of the present invention. When the content of the component (A1) is equal to or more than the lower limit and equal to or less than the upper limit, the effect of the present invention can be further enhanced. In particular, when the content of the component (A1) is equal to or less than the upper limit, generation of a precipitate in the antiperspirant composition can be effectively suppressed.

(Component (B))

The component (B) is aluminum chlorohydrate (aluminum hydroxychloride). The component (B) is an antiperspirant component. The component (B) is dissolved in an aqueous solvent, and the solution is applied to form a film after drying. The formed film blocks the perspiration glands to exhibit the antiperspirant effect. Only one kind of the component (B) may be used, or two or more kinds thereof may be used in combination.

From the viewpoint of exhibiting the effect of the present invention, the content of the component (B) is 3.0 mass% or more and 30.0 mass% or less per 100 mass% of the antiperspirant composition of the present invention.

The content of the component (B) is preferably 5.0 mass% or more, and more preferably 8.0 mass% or more, and preferably 25.0 mass% or less, and more preferably 20.0 mass% or less, per 100 mass% of the antiperspirant composition of the present invention. When the content of the component (B) is equal to or more than the lower limit and equal to or less than the upper limit, the effect of the present invention can be further enhanced. In particular, when the content of the component (B) is equal to or less than the upper limit, generation of a precipitate in the antiperspirant composition can be effectively suppressed.

(Component (C))

The component (C) is ethanol.

The content of the component (C) is preferably 1.0 mass% or more, more preferably 3.0 mass% or more, and still more preferably 4.0 mass% or more, and preferably 90.0 mass% or less, more preferably 85.0 mass% or less, and still more preferably 75.0 mass% or less, per 100 mass% of the antiperspirant composition of the present invention. When the content of the component (C) is equal to or more than the lower limit and equal to or less than the upper limit, the effect of the present invention can be further enhanced.

In the antiperspirant composition of the present invention, the mass ratio of the content of the component (C) to the content of the component (A) (content of component (C)/content of component (A)) is 20.0 or more from the viewpoint of exhibiting the effect of the present invention.

In the antiperspirant composition of the present invention, the mass ratio of the content of the component (C) to the content of the component (A) (content of component (C)/content of component (A)) is preferably 25.0 or more. When the mass ratio (content of component (C)/content of component (A)) is equal to or more than the lower limit, the effect of the present invention can be further enhanced. In particular, when the mass ratio (content of component (C)/content of component (A)) is equal to or more than the lower limit, generation of a precipitate in the antiperspirant composition can be effectively suppressed. The upper limit of the mass ratio of the content of the component (C) to the content of the component (A) (content of component (C)/content of component (A)) in the antiperspirant composition of the present invention is not particularly limited. The mass ratio of the content of the component (C) to the content of the component (A) (content of component (C)/content of component (A)) may be 500.0 or less.

(Component (D))

The component (D) is water. The component (D) is preferably purified water.

The content of the component (D) is preferably 8.0 mass% or more, more preferably 15.0 mass% or more, and still more preferably 20.0 mass% or more, and preferably 90.0 mass% or less, and more preferably 80.0 mass% or less, per 100 mass% of the antiperspirant composition of the present invention. When the content of the component (D) is equal to or more than the lower limit and equal to or less than the upper limit, the effect of the present invention can be further enhanced.

In the antiperspirant composition of the present invention, the mass ratio of the content of the component (D) to the content of the component (A) (content of component (D)/content of component (A)) is 10.0 or more from the viewpoint of exhibiting the effect of the present invention.

In the antiperspirant composition of the present invention, the mass ratio of the content of the component (D) to

the content of the component (A) (content of component (D)/content of component (A)) is preferably 15.0 or more, more preferably 30.0 or more, still more preferably 35.0 or more, and preferably 500.0 or less, more preferably 450.0 or less, still more preferably 400.0 or less. When the mass ratio (content of component (D)/content of component (A)) is equal to or more than the lower limit and equal to or less than the upper limit, the effect of the present invention can be further enhanced. In particular, when the mass ratio (content of component (D)/content of component (A)) is equal to or more than the lower limit, generation of a precipitate in the antiperspirant composition can be effectively suppressed.

In the antiperspirant composition of the present invention, the mass ratio of the content of the component (D) to the content of the component (B) (content of component (D)/content of component (B)) is 0.8 or more from the viewpoint of exhibiting the effect of the present invention.

In the antiperspirant composition of the present invention, the mass ratio of the content of the component (D) to the content of the component (B) (content of component (D)/content of component (B)) is preferably 1.0 or more, more preferably 1.2 or more, still more preferably 1.5 or more, and preferably 25.0 or less, more preferably 20.0 or less, still more preferably 15.0 or less. When the mass ratio (content of component (D)/content of component (B)) is equal to or more than the lower limit and equal to or less than the upper limit, the effect of the present invention can be further enhanced. In particular, when the mass ratio (content of component (D)/content of component (B)) is equal to or more than the lower limit, generation of a precipitate in the antiperspirant composition can be effectively suppressed.

(Other components)

The antiperspirant composition of the present invention may contain components other than the components (A) to (D) as long as the effect of the present invention is not impaired. Only one kind of each of the components other than the components (A) to (D) may be used, or two or more kinds thereof may be used in combination.

Examples of the other components include water-soluble polymers, ester oils, silicone oils, hydrocarbon oils, vegetable oils, bactericides, other antiperspirant components, refreshing agents, polyhydric alcohols, surfactants, humectants, deodorants, sequestering agents, preservatives, anti-inflammatory agents, plant extracts, powders, and fragrances.

Examples of the water-soluble polymer include acryl-based polymers such as carboxyvinyl polymers, alkyl-modified carboxyvinyl polymers, alkyl acrylate copolymers, (Acrylates/Steareth-20 Methacrylate) Copolymer, (Acrylates/Steareth-20 Methacrylate) Crosspolymer, (Acrylates/Beheneth-25 Methacrylate) Copolymer and (Acrylates/Vinyl Neodecanoate) Crosspolymer; Cellulose-based polymers such as methyl cellulose, ethyl cellulose, hydroxymethyl cellulose, hydroxyethyl cellulose, hydroxypropyl cellulose, carboxymethyl cellulose and hydroxypropyl methyl cellulose; and natural gum-based polymers such as xanthan gum, gelatin, guar gum, carrageenan, pectin and locust bean gum.

Examples of the ester oil include ethyl oleate, isopropyl myristate, isopropyl palmitate, myristyl myristate, cetyl palmitate, 2-ethylhexyl palmitate, octyldodecyl myristate, isopropyl isostearate, propylene glycol isostearate, cetyl 2-ethylhexanoate, glyceryl tri-2-ethylhexanoate, glyceryl tri(caprylate/caprate), isononyl isononanoate, diisopropyl adipate, pentaerythrityl tetraoctanoate, pentaerythritol tetraisostearate, diisostearyl malate, erythrityl triethylhexanoate, 2-ethylhexyl hydroxystearate, dipentaerythrityl hexa(hydroxystearate/stearate/rosinate), dipentaerythrityl tetra(hydroxystearate/isostearate), dipentaerythrityl hexahydroxystearate, glyceryl (ethylhexanoate/stearate/adipate), glyceryl tri(caprylate/caprate/myristate/stearate), and dipentaerythrityl hexa(behenate/benzoate/ethylhexanoate).

Examples of the silicone oil include dimethyl silicone oil such as methylpolysiloxane and high-polymerization methylpolysiloxane; methylphenyl silicone oil such as methylphenylpolysiloxane; cyclic silicone oil such as methylcyclopolysiloxane, octamethylcyclotetrasiloxane, decamethylcyclopentasiloxane and dodecamethylcyclohexasiloxane; modified silicone such as amino-modified silicone such as aminopropylmethylsiloxane/dimethylsiloxane copolymers, aminoethylaminopropylsiloxane/dimethylsiloxane copolymers and aminoethylaminopropylmethylsiloxane-dimethylsiloxane copolymers, carboxy-modified silicone, fatty acid-modified silicone, alcohol-modified silicone, aliphatic alcohol-modified silicone, epoxy-modified silicone, fluorine-modified silicone, and alkyl-modified silicone; and methylhydrogenpolysiloxane, and dimethiconol.

Examples of the hydrocarbon oil include $\alpha$-olefin oligomers, petrolatum, isoparaffin, light isoparaffin, light liquid isoparaffin, squalane, synthetic squalane, plant-derived squalane, liquid isoparaffin, liquid paraffin, hydrogenated polyisobutene, hydrogenated (tetradecenyl/methylpentadecene), and isododecane.

Examples of the vegetable oil include macadamia nut oil, eucalyptus oil, coconut oil, avocado oil, safflower oil, olive oil, palm oil, palm kernel oil, candlenut oil, shea fat (shea butter), cacao butter, almond oil, sunflower oil, rose hip oil, olive squalane, camellia oil, kiwifruit seed oil, tea seed oil, apricot kernel oil, sesame oil, soybean oil, jojoba oil, castor oil, hazelnut oil, meadowfoam oil, mentha oil, argan oil, carrot oil, lavender oil, sugar squalane, damask rose wax, centifolia rose wax, osmanthus flower wax, and hydrogenated products thereof (e.g., hydrogenated castor oil, hydrogenated jojoba oil, hydrogenated palm oil, hydrogenated avocado oil and hydrogenated soybean oil).

Examples of the bactericide include isopropylmethylphenol, benzalkonium chloride, hinokitiol, and salicylic acid.

Examples of the refreshing agent include menthol, camphor, and menthyl glyceryl ether.

Examples of the polyhydric alcohol include ethylene glycol, propylene glycol, dipropylene glycol, polyethylene glycol, 1,3-butylene glycol, glycerin, concentrated glycerin, diglycerin, triglycerin, glucose, maltose, maltitol, sucrose, mannitol, sorbitol, 1,2-pentanediol, 1,2-hexanediol, and 1,2-octanediol.

(Other details of antiperspirant composition)

The dosage form of the antiperspirant composition of the present invention is not particularly limited. Examples of the dosage form of the antiperspirant composition of the present invention include an aerosol, a lotion, a mist, an emulsion, a gel, a cream, a roll-on, a stick, and a sheet.
The antiperspirant composition of the present invention can be produced by a known conventional method for producing an antiperspirant composition. The antiperspirant composition of the present invention can be produced by, for example, mixing the component (A), the component (B), the component (C), the component (D), and other components blended if necessary. The respective components can be mixed using a disperser mixer or the like.

[Antiperspirant]

An antiperspirant includes a container and the above-described antiperspirant composition. In the antiperspirant, the container is filled with the antiperspirant composition.
The container is preferably a roll-on container. The antiperspirant is preferably used as a roll-on-type antiperspirant.
The roll-on container includes a roll in an applicator. The roll may be a cylindrical member, or a spherical member.
In the roll-on-type antiperspirant, the antiperspirant composition can be directly and uniformly applied to an object to be coated, such as the skin without putting the antiperspirant composition on a hand for application to the object to be coated. Therefore, the roll-on type antiperspirant is excellent in applicability. In the roll-on type antiperspirant, the antiperspirant composition in the roll-on container can be easily and directly applied to the skin of, for example, the armpit.
The antiperspirant can be preferably used not only as the roll-on-type antiperspirant but also as an antiperspirant of aerosol type, lotion type, mist type, emulsion type, gel type, cream type, stick type, sheet type or the like. In this case, the antiperspirant composition of the present invention is filled into a container suitable for use with a relevant type of antiperspirant.

EXAMPLES

Hereinafter, the present invention will be described in detail by way of examples and comparative examples. The present invention is not limited to the following examples.
In examples and comparative examples, the following components were used.

(Component (A))
Monoammonium glycyrrhizate
(Component (B))
Aluminum chlorohydrate
(Component (C))
Ethanol
(Component (D))
Purified water

(Others)

Zinc paraphenolsulfonate
Glyceryl tri-2-ethylhexanoate (ester oil)
Polyethylene glycol 400 (polyhydric alcohol)
Hydroxypropyl cellulose (water-soluble polymer)

(Examples 1 to 9 and Comparative Examples 1 to 11)

The blending components shown in Tables 1 and 2 below were blended (in a unit of mass%) to prepare antiperspirant compositions shown in Tables 1 and 2 below. In the tables, the blending amount (blending amount per 100

mass% of the antiperspirant composition) is shown as a blending amount of a pure component (unit: mass%).

(Evaluation)

In the following test examples, three evaluation panels engaged in evaluation.

(Test Example 1: Precipitate)

The properties of the antiperspirant composition immediately after preparation were visually observed.

<Evaluation criteria for precipitate>

◦ (Good): A precipitate is not observed.
× (Poor): A precipitate is observed.

For a composition which was poor in the result of Test Example 1 (a composition in which a precipitate was observed), evaluation in "Test Example 2: Uniformity of film" below was not performed ("-" is written in the tables") because a stable composition was not formed.

(Test Example 2: Uniformity of film)

The obtained antiperspirant composition was dropped in an amount of 100 μL onto a surface of an artificial skin ("BIO SKIN PLATE" manufactured by Beaulax), uniformly stretched, and dried. The state of the film after drying was visually observed. The uniformity of the film was evaluated on the basis of the following evaluation criteria.

<Evaluation criteria for uniformity of film>

@ (Good): The film is not cracked, and a uniform film is formed.
◦ (Fair): The film has slight unevenness, but is not cracked, and a uniform film is formed.
× (Poor): The film is cracked, and a uniform film is not formed.

(Test Example 3: Antiperspirant effect)

For the antiperspirant compositions obtained in Example 9 and Comparative Example 11, the amount of perspiration was measured by the following procedure. The evaluation was performed on three subjects.

The obtained antiperspirant composition was applied to the lower armpit of a subject, and dried. Subsequently, the subjects engaged in bicycle pedaling exercise in an environment at a temperature of 30°C and a humidity of 60% to induce perspiration. Using a sweating meter ("SKN-2000" manufactured by Nishizawa Electric Meters Manufacturing Co., Ltd.), the amount of perspiration (mg) at an antiperspirant composition-applied area (measurement area: 1cm$^2$) 30 minutes after the start of bicycle pedaling exercise was measured. The perspiration inhibition ratio in Example 9 with respect to that in Comparative Example 11 was calculated from the following equation.

Perspiration inhibition ratio (%) = 100 - [(amount of perspiration in Example 9/amount of perspiration in Comparative Example 11) × 100]

The result showed that the perspiration inhibition ratio in Example 9 with respect to that in Comparative Example 11 was 40.5%, and the perspiration was inhibited to a higher degree in Example 9 than in Comparative Example 11.

Compositions and results are shown in Tables 1 and 2 below.

[Table 1]

| | | | Example | | | | | | | | |
|---|---|---|---|---|---|---|---|---|---|---|---|
| | | | 1 | 2 | 3 | 4 | 5 | 6 | 7 | 8 | 9 |
| Component (A) | Monoammonium glycyrrhizate | mass% | 0.20 | 0.90 | 0.20 | 0.20 | 0.05 | 0.20 | 0.90 | 0.20 | 0.30 |
| Component (B) | Aluminum chlorohydrate | mass% | 25.0 | 20.0 | 20.0 | 10.0 | 20.0 | 5.0 | 4.0 | 10.0 | 10.0 |
| | Zinc paraphenolsulfonate | mass% | | | | | | | | | |
| Component (C) | Ethanol | mass% | 40.0 | 18.0 | 4.0 | 70.0 | 1.0 | 4.0 | 86.1 | 81.8 | 70.0 |
| Component (D) | Purified water | mass% | 34.8 | 61.1 | 75.8 | 17.8 | 78.9 | 90.8 | 9.0 | 8.0 | 19.7 |
| | Glyceryl tri-2-ethylhexanoate | mass% | | | | 0.5 | | | | | |
| | Polyethylene glycol 400 (PEG-400) | mass% | | | | 1.0 | | | | | |
| | Hydroxypropyl cellulose | mass% | | | | 0.5 | | | | | |
| Total | | mass% | 100.0 | 100.0 | 100.0 | 100.0 | 100.0 | 100.0 | 100.0 | 100.0 | 100.0 |
| Mass ratio (content of component (C)/ content of component (A)) | | | 200.0 | 20.0 | 20.0 | 350.0 | 20.0 | 20.0 | 95.7 | 409.0 | 233.3 |
| Mass ratio (content of component (D)/ content of component (A)) | | | 174.0 | 67.9 | 379.0 | 89.0 | 1578.0 | 454.0 | 10.0 | 40.0 | 65.7 |
| Mass ratio (content of component (D)/ content of component (B)) | | | 1.4 | 3.1 | 3.8 | 1.8 | 3.9 | 18.2 | 2.3 | 0.8 | 2.0 |
| Evaluation | Precipitate | | ○ | ○ | ○ | ○ | ○ | ○ | ○ | ○ | ○ |
| | Uniformity of film | | ⊙ | ⊙ | ⊙ | ⊙ | ○ | ○ | ○ | ⊙ | ⊙ |

[Table 2]

| | | | Comparative Example | | | | | | | | | | |
|---|---|---|---|---|---|---|---|---|---|---|---|---|---|
| | | | 1 | 2 | 3 | 4 | 5 | 6 | 7 | 8 | 9 | 10 | 11 |
| Component (A) | Monoammonium glycyr-rhizate | mass% | 0.90 | 0.20 | 0.20 | 0.01 | 1.20 | 0.20 | 0.90 | 0.40 | 0.20 | | |
| Component (B) | Aluminum chlorohydrate | mass% | 1.0 | 20.0 | 35.0 | 25.0 | 20.0 | 20.0 | 3.0 | 20.0 | | 20.0 | 10.0 |
| | Zinc paraphenolsulfonate | mass% | | | | | | | | | 0.3 | 0.3 | |
| Component (C) | Ethanol | mass% | 18.0 | 3.5 | 4.0 | 4.0 | 24.0 | 2.0 | 90.1 | 69.6 | 4.0 | 4.0 | 70.0 |
| Component (D) | Purified water | mass% | 80.1 | 76.3 | 60.8 | 71.0 | 54.8 | 77.8 | 6.0 | 10.0 | 95.5 | 75.7 | 20.0 |
| | Glyceryl tri-2-ethylhex-anoate | mass% | | | | | | | | | | | |
| | Polyethylene glycol 400 (PEG-400) | mass% | | | | | | | | | | | |
| | Hydroxypropyl cellulose | mass% | | | | | | | | | | | |
| Total | | mass% | 100.0 | 100.0 | 100.0 | 100.0 | 100.0 | 100.0 | 100.0 | 100.0 | 100.0 | 100.0 | 100.0 |
| Mass ratio (content of component (C)/ content of component (A)) | | | 20.0 | 17.5 | 20.0 | 400.0 | 20.0 | 10.0 | 100.1 | 174.0 | 20.0 | - | - |
| Mass ratio (content of component (D)/ content of component (A)) | | | 89.0 | 381.5 | 304.0 | 7100.0 | 45.7 | 389.0 | 6.7 | 25.0 | 477.5 | - | - |
| Mass ratio (content of component (D)/ content of component (B)) | | | 80.1 | 3.8 | 1.7 | 2.8 | 2.7 | 3.9 | 2.0 | 0.5 | - | 3.8 | 2.0 |
| Evaluation | Precipitate | | ○ | × | × | ○ | × | × | × | × | ○ | ○ | ○ |
| | Uniformity of film | | × | - | - | × | - | - | - | - | × | × | × |

## Claims

**1.** An antiperspirant composition comprising a component A, a component B, a component C, and a component D,

wherein a content of the component A is 0.05 mass% or more and 1.0 mass% or less,
a content of the component B is 3.0 mass% or more and 30.0 mass% or less,
a mass ratio of the content of the component C to the content of the component A is 20.0 or more,
a mass ratio of the content of the component D to the content of the component A is 10.0 or more, and
a mass ratio of the content of the component D to the content of the component B is 0.8 or more:

the component A: ammonium glycyrrhizate
the component B: aluminum chlorohydrate
the component C: ethanol
the component D: water.

**INTERNATIONAL SEARCH REPORT**

International application No. **PCT/JP2024/033226**

**A. CLASSIFICATION OF SUBJECT MATTER**

***A61K 8/26***(2006.01)i; ***A61K 8/34***(2006.01)i; ***A61K 8/63***(2006.01)i; ***A61Q 15/00***(2006.01)i
FI: A61K8/26; A61K8/63; A61K8/34; A61Q15/00

According to International Patent Classification (IPC) or to both national classification and IPC

**B. FIELDS SEARCHED**

Minimum documentation searched (classification system followed by classification symbols)

A61K8/26; A61K8/34; A61K8/63; A61Q15/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

Mintel GNPD

**C. DOCUMENTS CONSIDERED TO BE RELEVANT**

| Category* | Citation of document, with indication, where appropriate, of the relevant passages | Relevant to claim No. |
|---|---|---|
| Y | CN 102415953 A (DONG, Lixia) 18 April 2012 (2012-04-18) paragraphs [0001]-[0009], [0019]-[0020], [0035]-[0052] | 1 |
| Y | JP 2021-46382 A (MANDOM CORPORATION) 25 March 2021 (2021-03-25) paragraphs [0001]-[0006], [0031]-[0035], table 1, examples 1-4, comparative example 2, etc. | 1 |
| A | JP 2020-125291 A (ROHTO PHARMACEUT CO., LTD.) 20 August 2020 (2020-08-20) | 1 |
| A | JP 2018-203683 A (LION CORPORATION) 27 December 2018 (2018-12-27) | 1 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 November 2024** | **26 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/033226**

| Patent document cited in search report | Publication date (day/month/year) | Patent family member(s) | Publication date (day/month/year) |
|---|---|---|---|
| CN 102415953 A | 18 April 2012 | (Family: none) | |
| JP 2021-46382 A | 25 March 2021 | JP 2023-168621 A | |
| JP 2020-125291 A | 20 August 2020 | (Family: none) | |
| JP 2018-203683 A | 27 December 2018 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003095906 A